# EUROPEAN PATENT SPECIFICATION

(11) **EP 2 356 249 B1**
(45) Date of publication and mention of the grant of the patent: **17.02.2016**
(21) Application number: 09740087.3
(22) Date of filing: 09.10.2009
(51) Int. Cl.: C12Q 1/68, C12M 3/00

(54) **GENETIC ANALYSIS IN MICROWELLS**
GENETISCHE ANALYSE IN MIKROVERTIEFUNGEN
ANALYSE GÉNÉTIQUE DANS DES MICROPUITS

(30) Priority: 10.10.2008 SE 0802171
(43) Date of publication of application: 17.08.2011
(73) Proprietor: Picovitro Ab, 553 03 Jönköping (SE)
(72) Inventor: ANDERSSON SVAHN, Hélène, S-191 38 Sollentuna (SE); AFSHIN, Ahmadian, S-113 57 Stockholm (SE); LINDSTRÖM, Sara, S-113 60 Stockholm (SE)
(74) Representative: Awapatent AB
(86) International application number: PCT/EP2009/063187
(87) International publication number: WO 2010/040831

(56) References cited:
- WO-A1-2009/021215
- WO-A1-2009/123748
- WO-A2-2007/147079
- US-A1- 2002 072 116
- LINDSTROEM SARA ET AL: "Towards high-throughput single cell/clone cultivation and analysis" ELECTROPHORESIS, vol. 29, no. 6, March 2008 (2008-03), pages 1219-1227, XP002561034 ISSN: 0173-0835
- MARCY Y ET AL: "Nanoliter reactors improve multiple displacement amplification of genomes from single cells" PLOS GENETICS 200709 US, vol. 3, no. 9, September 2007 (2007-09), pages 1702-1708, XP002561035
- THORSEN TODD ET AL: "Microfluidic large-scale integration." SCIENCE (NEW YORK, N.Y.) 18 OCT 2002, vol. 298, no. 5593, 18 October 2002 (2002-10-18), pages 580-584, XP002561036 ISSN: 1095-9203
- DATABASE BIOSIS [Online] BIOSCIENCES INFORMATION SERVICE, PHILADELPHIA, PA, US; 16 November 2002 (2002-11-16), SHIN MYUNG GEUN ET AL: "Unexpected Mitochondrial DNA Heterogeneity in Single CD34+ Cell Clones from Normal Bone Marrow." XP002561037 Database accession no. PREV200300367589
- TOKIMITSU YOSHIHARU ET AL: "Single lymphocyte analysis with a microwell array chip." CYTOMETRY. PART A : THE JOURNAL OF THE INTERNATIONAL SOCIETY FOR ANALYTICAL CYTOLOGY DEC 2007, vol. 71, no. 12, December 2007 (2007-12), pages 1003-1010, XP002561038 ISSN: 1552-4930
- TSUCHIYA ET AL: "On-chip polymerase chain reaction microdevice employing a magnetic droplet-manipulation system", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 130, no. 2, 18 October 2007 (2007-10-18), pages 583-588, XP022550371, ISSN: 0925-4005
- WATERS L C ET AL: "MICROCHIP DEVICE FOR CELL LYSIS, MULTIPLEX PCR AMPLIFICATION, AND ELECTROPHORETIC SIZING", ANALYTICAL CHEMISTRY, AMERICAN CHEMICAL SOCIETY, US, vol. 70, no. 1, 1 January 1998 (1998-01-01), pages 158-162, XP000733220, ISSN: 0003-2700, DOI: 10.1021/AC970642D

## Description

### Field of the invention

The present invention relates to a method of genetic analysis of single cell samples, such as one single cell and clones expanded from one single cell. Disclosed is a method of cell seeding, wherein at least one cell is seeded into a microplate comprising a high number of wells/cm², as well as to a method of cell cultivation, wherein at least one cell is seeded, cultivated and analyzed in a microplate.

### Background

Methods and platforms for advanced cellomics are important areas of research as the minimum unit of a living system is a single cell. The living human cell is also the ultimate target of all drugs. To fully understand human cells and their biological response to drug therapy, the complex pathways involved in all cellular functions need to be interpreted. Powerful tools for detailed cellular studies are emerging, making it possible to learn more about cell biology. High confidence is put in advanced micro- and nanotechnological tools that, combined with living cells, create so called laboratory-in-a-cell (LIC) and constitute promising approaches for cell access and analysis.

Because of the heterogeneity within a cell population, increased emphasis has been put on analyzing a large number of individual cells and determining the distributions of responses (Mettetal, et al, Proc Natl Acad Sci U S A 103, 7304-7309 (2006)). Even in genetically identical populations, phenotypic and behavioral cell-to-cell variations have since long been observed.

A fundamental goal of cell biology is thus to quantify the range of biological responses of individual cells to various physiologically relevant stimuli, as opposed to bulk averages (e.g. Lidstrom et al, Nat Rev Microbiol 1, 158-164 (2003)).

Several approaches to single cell analysis have been presented, some of which are accounted for below.

### Single cell analysis

One widely used method of ordering large numbers of single cells into arrays is by micropatterning of surfaces and seeding cells on these controlled patches of extra cellular matrix (ECM) (Kane et al, Biomaterials 20, 2363-2376 (1999)).

The micropatterning approaches, however, are associated with a number of drawbacks. The drawbacks are, among others, disruptive cell tethering and changed cell behavior, the latter limiting the possibilities for long-term analysis. Thus, only short-term analysis is possible. In addition, these approaches often demand adherent cells for successful seeding. Furthermore, several cells instead of single cells easily become attached per micropatterned spot. The usefulness of the micropatterning approaches is consequently limited.

In addition, many attempts to achieve single cell analysis necessarily involve treating all cells in the same manner. This is often the case for microfluidic systems. Moreover, microfluidic systems and similar fluidic devices often give rise to shear stresses, which might decrease the proliferation rate for sensitive cells. This implies a need for alternative methods of analysis which offer the possibility of treating single cells individually.

For some types of single cell analysis, and e.g. cell-to-cell interaction studies, it is important to know the specific position in an analytical system for a specific cell. A specific cell has to be assigned to a specific position, and that specific position and the specific cell have to be addressable later. However, most work on single cell handling techniques involves random positioning of cells, meaning that the cells adhere at any place in the analytical system by simply settling down and without possibility to track that single cell over time. Preparing the right dilution of the cell sample then becomes critical to give the highest possible single cell occupancy, for example in microwells or on patterned surfaces.

There are several ways of studying single cells. Common ways of studying single cells are different image-based techniques using a microscope often in combination with various intracellular fluorescent probes that have been used both for cells grown on conventional devices and in microsystems. Conventional systems for single cell analysis include capillary electrophoresis (CE) and flow cytometry.

Using flow cytometry, cells in solution can be analyzed according to cell concentration, size, shape, granularity, protein expression levels, DNA contents etc (Akerlund et al, J Bacteriol 177, 6791-6797 (1995)). Hundreds of thousands of cells can be analyzed at a single cell level for up to 16 parameters. Time-dependent analysis, in which different cells are sampled at each time point for several minutes, can also be conducted. Although flow cytometry is a very high throughput system there are some drawbacks. Flow cytometry is a very useful method to detect whether and in what amount a certain molecule is present but unlike microscope based techniques the spatial localization cannot be observed. It is not possible to follow the cells over time by flow cytometry; neither to track the dynamics of individual cells nor to identify specific cells after cell division. The fact that the cells are in a flow when analyzed means that, since most cells are adherent cells, the cells cannot be analyzed in their natural environment. Flow cytometry, however, reveals limited information on cell content and gives no information on cell proliferation and clone formation capabilities of a cell sample. In addition, although flow cytometry enables analysis of single cells in a group, group average data is obtained instead of individual cell response data.

EP79528 discloses a method for selecting desirable cell clones, which comprises automated cell seeding using a flow cytophotometer. The method comprises adding cultivation media to a microtiter plate, adding single cells to each well of the plate, incubating the microtiter plate, and analyzing the filtrate from the cultivation media for metabolites. The method primarily involves cultivation of prokaryotic cells. It furthermore has low throughput, presumably due to the low number of wells in the plate and the large volumes involved.

Flow cytometry generally provides information about the average properties of cells, revealing how a group of cells changes. Dynamic information on the level of single cells is however not available. Dynamic information requires continuous tracking of the dynamics of specific cells. However, as flow cytometry is an instant method, the sample withdrawn from culture is often discarded after measurement. Flow cytometry thus lacks means for keeping cells in a uniform environment for long-term monitoring. Moreover, it cannot identify a particular cell, especially not after cell division has occurred.

Strategies for acquiring dynamic information without the use of flow cytometry have recently been presented. One example is disclosed in Di Carlo et al, wherein a dynamic single cell culture array with U-shaped trapping structures is presented. The array allows for cultivation of individual adherent cells in a uniform environment (Di Carlo, et al, Lab Chip 6, 1445-1449 (2006)). Cell cultivation in this and other similar devices is however often limited to a few hours of cultivation, or at best to one or two days. Such a relatively short time frame will restrict the cell passages to 1-2 cell generations, which consequently might limit the usefulness of the method. Moreover, non-adherent cells, e.g. blood cells, can be more difficult to study in such an array.

Single cells could be seeded into 96-well plates but considering the size of a cell is in the order of tens of microns in diameter and the volume is only about 1 pl a single cell would almost disappear in these sizes of wells. A microfabricated device would thus be a great benefit for single cell analysis because of its small volume.

There are several ways to arrange cells on microplates. Cells can be arranged on microfluidic microplates where an intricate system of valves and sorting mechanisms can trap cells in different compartments where buffer can easily be exchanged in a rapid and controlled way. The flow in these devices is very useful to mediate transport but it is also a disadvantage as it might create shear stresses on the cells that will inevitably affect the metabolic function and morphology of the cells (Tanaka, et al, Biosens Bioelectron 23, 449-58 (2007)).

The interaction between adherent cells and extracellular matrix (ECM) can also be used to organize cells on arrays by spotting ECM components in individual spots where only one cell will fit. There are also reports of spotting single stranded (ss) DNA on arrays and labeling cells with the complementary strand. The cells can then be bound to the array by DNA hybridization. Cells can also be organized in microwell arrays. One common way to make sure only one single cell will be present in each well is to design the wells to only fit one cell.

Cultivation of eukaryotic cells has for a long time been a commonly used tool in biomedical research. Cell cultures are an easy and inexpensive way of studying biological processes. It could be debated how well a cell in a cell culture in a Petri dish mimics a cell in a complex organ in a complex body. What should also be considered regarding cell cultures, but also patient samples (blood and tissue), is the accuracy of studying cells in bulk as is commonly done.

If cells were studied in bulk, two populations (Di Carlo, et al, Anal Chem 78, 7918-25 (2006)) would not necessarily be noticed as two distinct populations but rather as one with a response that does not match the true responses of any of the cells. If the cells were studied in bulk, it would look like they had a somewhat linear increase in their response while the single-cell dynamics show an on-off behavior where a single cell goes from an off-state to an on-state in a very short time. This on-off behavior is believed to be very common for many signal transducers. When studying bulk cell samples, it might also be difficult to distinguish if two genes are co-expressed in the same cell or if there are two different cell populations; one expressing one gene and one expressing the other (Sims, Lab Chip 7, 423-40 (2007)).

A good example where studies would benefit from studying single cells instead of cells in bulk is when studying primary cells from patients. It is known that a tumor sample contains a lot of cells that are not diseased but also that the abnormal cells in the sample differ in contrast to each other. Cell lines mostly originate from one single cell and all cells are thus expected to be identical. Cell lines might therefore not be benefiting so much from single cell studies but rather be used for proof of concept studies during methods development. What should not be overlooked is that when cultivating cells in larger devices there will be heterogeneity in the environment considering diffusible secretions and the possibilities for different cells at different locations in the culture to form cell-cell contacts so even cell lines could possibly benefit from being analyzed at a single cell level after cultivation in bulk.

What is common for many of these array approaches is that the cells cannot be single cell seeded and cultivated for a longer period of time since there are space limitations only allowing one cell at the same spot and thereby inhibiting cell proliferation or if a larger well/spot is allowed more then one cell can be seeded in that location.

Heterogeneity marks a current paradigm shift in clinical diagnostics, where correlation of proliferation screening, cell morphology, proteomic and genetic analysis is of great interest. When tumor biopsies are screened for mutations, a major problem is the overshadowing effect by normal or non-mutated cells. However, as today's available methods are not able to detect mutation frequencies of less than 10%, a method where mutations can be detected independently of the frequency of normal cells is needed.

In order to understand cellular processes and behavior, a controlled way of studying high numbers of single cells and their clone formation is greatly needed. Numerous ways of ordering single cells into arrays have previously been described WO-A-2007/147079 discloses a procedure in which single cells are sorted into individual wells. A microplate having microwells wherein the radius between the centres of two wells is about 35 micrometers is suggested. PCR primers may be added to each individual well. The amplicons may be labelled with a MIP which identify the SNP amplified and the well. LINDSTROEM S ET AL: "Towards high-throughput single cell/clone cultivation and analysis", ELECTROPHORESIS, vol. 29(6), 2008, pages 1219-1227 discloses a microplate. Single cells are deposited and cultivated for up to 14 days in the same media in the wells. The volume of each well is 500 nl and the wells have tilted walls. The seeding is performed by FACS. The cells used in the examples are leukemia cells and myeloma cells. However, methods wherein each cell and/or clone can be directed and continuously addressed to an exact position, cultivated for weeks, treated differently and genetically analysed in a high-throughput manner have not been previously described. Accordingly, to progress from temporal observation of a group of cells giving a group average as result, biology needs more single-object approaches. **Description of the invention**

Disclosed is a method of controlled cell seeding, wherein from a cell sample a large number of single cells can be seeded into wells of a microplate in a highly reproducible and high-throughput manner. Disclosed are further methods of cell cultivation, wherein single cells from a cell sample can be seeded into wells of a microplate and cultivated, enabling short- or long-term study and treatment of cells on the single cell level.

An object of the invention is to provide methods of genetic analysis of single cell samples contained in a microplate, enabling genetic analysis of at least one single cell and clones expanded from at least one single cell.

Thus, disclosed is a method of cell seeding comprising the steps of:
- providing a cell sample;
- seeding at least one cell of said cell sample into at least one well of a microplate comprising at least 5 wells/cm².

Consequently, the method of cell seeding enables seeding of one or more cells into one or more wells of a microplate having a large well density. In particular, the method enables seeding of single cells into discrete wells, i.e. at least one single cell is seeded into at least one well.

For example, microplates comprising 5 wells/cm²-700 000 wells/cm² can be used with the method. The microplate format and dimension can be any desired format or dimension, provided that the number of wells per cm² is at least 5. If, for example, a microplate with dimensions of a standard 96-well plate is used (128 mm in length and 86 mm in width and with an available well area of 76 cm²), the microplate can comprise from 380 wells to 53 million wells, such as 500-8.5 million wells, such as 1000-100 000 wells, and such as 3000-10 000 wells. Another possible microplate format is represented by the array format, i.e. 76 mm in length and 26 mm in width. If a microplate of array format is used, it can comprise from 91 wells to 12.7 million wells, such as from 91 wells to 2.1 million wells, and such as 500-10 000 wells.

The method enables high-throughput and controlled cell seeding. In this context, high-throughput seeding could for example mean that thousands of single cells can be seeded into discrete wells. Controlled seeding implies that specific single cells are assigned to specific wells and that those specific cells are addressable later. Growth medium may be added to all desired wells simultaneously, prior to cell seeding.

The seeding method can for example be performed using any automatic robot equipment.

Alternatively, manual seeding by limited dilution may be performed, e.g. by allowing an estimated number of cells to randomly sit down in the different microwells.

The seeding method can for example be performed using a flow cytometer apparatus, for example a flow cytometer comprising sorting means. By using flow cytometry for cell seeding, the output can be maximized and a high number of cells can be seeded rapidly and with high accuracy. This means that the time needed for seeding a number of single cells into specific wells is very low. In addition, single cell seeding may be performed with a low margin of error. Thus, single cells will indeed be seeded into a majority of the wells assigned for seeding.

The method can be performed using a type of flow cytometry apparatus known as a fluorescence activated cell sorter (FACS). For example, if cell seeding is performed using a FACS, e.g. a FACSVantage SE Cell Sorter (BD Biosciences), the time needed for seeding of single cells into wells may be one second per cell and well. Furthermore, the desired single cell seeding may be attained for more than 80% of the assigned wells. The use of such an established cell-sorter and analysis instrument provides distinct advantages, including high sensitivity and accuracy. One advantage of using FACS is the possibility of analyzing cells while they are seeded into the wells and maintaining the possibility for further high-throughput analysis during long time periods.

Disclosed is a method of cell cultivation comprising the steps of:
- seeding at least one cell into a microplate using the seeding method as disclosed herein;
- incubating the microplate; and
- analyzing the contents of at least one well of the microplate.

Optional features of the seeding step are as defined above in connection with the description of the seeding method, for example with regard to microplate dimensions and other parameters.

The method of cultivation thus provides rapid and controlled cell seeding, incubation of cells contained in the microplate and further downstream analysis in-plate. Analysis may thus be performed for thousands of single cells contained in discrete wells in parallel.

In the method of cultivation, seeding may be performed first and the other steps may be performed in any order. Moreover, at least two of the steps of seeding, incubation and analysis may be performed simultaneously. In particular, analysis may be performed while seeding.

At least one of the steps of incubation and analysis may be repeated at least once.

The method of cultivation allows any desired number of cells, for example several thousands of single cells, to be cultivated (or incubated) for any desired time period. Cells can for example be cultivated and continually monitored and analyzed for one month. By seeding single cells into single wells of the microplate, cells can for example be analyzed individually for over 14 generations, ending up with more than 10 000 cells in each well.

Analysis of well contents can be performed directly after cell seeding or at any desired point of time, during incubation or after incubation, and can be repeated at any time. Any standard detection equipment can be used in the analytic step of the method of cultivation. A microscope of any kind (e.g. confocal microscope) can for example be used for manual detection either well by well or several wells at a time in one picture. The number of wells detected in one picture depends on the magnification/object being used. A microscope with a programmable x/y-stage can also be used for automatic detection, for example when analyzing fluorescently labeled cells. Automatic detection here means that a software runs the x/y-stage that moves the plate/array back and forth, which enables photographing (i.e. detection) well by well in high-throughput. An array scanner is another example of a type of detection equipment that can be used in the method. All kinds of fluorescent plate readers can furthermore be used, provided that the point of detection (coordinates in x/y directions) can be controlled (for well by well detection) or run in a scanning mode (for scanning of the whole plate area regardless of the number of wells). It is understood that other types of detection equipment than the types mentioned above can be used in the method.

Analysis of well contents may for example comprise analysis of number of cells, cell appearance, analysis of cell contents, cell products, protein expression, nucleic acids, etc.

The cultivation method enables easy location of cells after clone formation, or at any desired point in time, because of the possibility of controlled assignment of specific cells to specific wells. This allows for a possibility of collecting dynamic information of the seeded cells. One can, for example, conclude on cells' abilities and heterogeneity in proliferation. Cells' abilities to proliferate, sometimes under the influence of affecting drugs, is a tremendously important aspect in life science and drug development, regardless of whether the objects of study are cancer cells, stem cells or other cells.

The methods using a microplate provide maintenance of a high cell retention during cultivation and analysis. Problems regarding maintenance of single cells in their original positions of trapping have been discussed earlier, for example in Di Carlo et al, Lab Chip (2006) (*supra*)*.* Reliability is an important characteristic for any method of cell seeding and cultivation. The method of cultivation thus accomplishes a high degree of reliability in that the method assures that the same single cells in the same wells are studied at different points in time.

In one example of the method of cultivation, analytic medium is added to at least one well at any point after the seeding step. In this example, at least one of the steps of incubation, analysis and addition may be repeated at least once. Analytic medium can for example be added before incubation, or at any point of incubation, before or after analysis. Analytic medium can be added at several points during performance of the method and addition can be repeated.

The term "analytic medium" refers to any substance or matter which can be added to at least one well of a microplate in any form. By way of non-limiting example, the analytic medium may comprise chemotherapeutic compounds; proteins; peptides; antibodies; affinity compounds; labeled compounds, for example labeled antibodies or labeled affinity compounds; nucleic acids; particles, such as for example magnetic beads; other compounds having or suspected of having a biological effect (e.g. drugs or drug leads), etc.

Analytic medium can for example be added using an automatic robot equipment. In particular, addition of analytic medium can be performed using a flow cytometer apparatus, such as for example a FACS. A flow cytometer apparatus, such as for example a FACS, may be used for addition of, for example, chemotherapeutic drugs to the wells of a microplate to enable study of cell/clone response to different drugs and drug concentrations in a high-throughput manner. This addition can for example be accomplished by running the flow cytometer in special mode, wherein artificial cells are registered as events which can be sorted out from the flow. A more suitable instrument of choice would be a standard nanoliter liquid dispenser (available for example from LabCyte, Perkin-Elmer and compatible with the described microplate). Automatic liquid dispensers have good compatibility with high-throughput microwell plates.

In the context of the present disclosure, the term "microplate" refers to any substantially planar arrangement of discrete wells or surfaces that can be used for cell cultivation. Microplate refers to, for example, analytical plates, microtiter plates, 96-well plates, multiwell plates, arrays, disks, or other plates used in biology, chemistry and related disciplines. One example of a microplate is disclosed in US patent no. 6,037,171.

One example of a microplate for use in the methods according to the invention comprises wells having tilted walls. Tilted well walls in this context mean that the well opening area is larger than the well bottom area. The well walls thus form an acute angle with a plane parallel to the microplate bottom. For example, the walls are tilted with an angle within the range of 20-85°, such as for example 40-65°, in particular 50-60°. One example of a microplate has walls that are tilted at an angle of 54.7°. Tilted walls facilitate seeding of one or more cells, whereby the method of seeding provides improved reproducibility and reliability compared to previously known methods.

If, for example, a microplate with well walls tilted at an angle of 54.7° is used, the well density may be from 5 wells/cm² to 112 000 wells/cm². If for example the microplate with tilted walls as above further has the dimensions of a standard 96-well plate (128 mm x 86 mm), the microplate can comprise from 380 wells to 8.5 million wells, such as 500-100 000 wells, such as 1000-10 000 wells, and such as 3000-10 000 wells.

Another example of a microplate for use in the methods according to the invention comprises wells having straight walls, i.e. the angle between the well walls and a plane parallel to the microplate bottom is 90°. Straight well walls enables a microplate comprising a higher well density compared to a microplate having tilted walls. A microplate having straight well walls can for example comprise 5-700 000 wells/cm².

The microplate may be thin and flat, i.e. the microplate thickness is low and the microplate is planar. The microplate thickness may for example be within the range of 400-2000 µm. One example of a microplate for use in the inventive methods has a plate thickness of 1000 µm. Other examples of microplates have thicknesses of 675 µm and 1500 µm. Low plate height provides for lower volume per well and improved detection and imaging when analyzing well contents, for example the possibility of coming closer to the sample with an objective in both upright and inverted microscopy. The center-to-center distance between wells in the microplate describes the closeness of the centers of two adjacent wells. The smaller the center-to-center distance, the larger the number of wells in the microplate. The center-to-center distance of wells may for example be within the range of 12-1500 µm, such as for example 30-1500 µm, and such as 900-1500 µm.

The methods provide seeding and cultivation in wells of a microplate. The wells of the microplate may be small, a single well for example comprising a volume from 1 pl to 200 µl, such as from 2 pl to 2000 nl, and such as from 150-500 nl. The wells may have any shape, for example circular shape, angular shape, etc. The wells may be square-shaped.
Then the well size, i.e. the well sidewall, may be from 10 µm to 4000 µm, such as for example 20-640 µm, and such as for example 100-500 µm. The wells may thus be optimized for single cell seeding, single cell culture, and single cell detection, since the wells may provide an appropriate volume of growth medium for single cell cultivation as well as a suitable well size for single cell detection.

For seeding and studying less than 1000 cells in the methods, a microplate of array format (76 mm x 26 mm) may be used. The microplate format may be adjusted to the amount of cells that will be seeded and studied as well as to the desired detection method. The array format is also a standard format for analysis and detection in for example microscope stages, flow cytometer x/y-stage plate holders, scanners etc. Furthermore, it is possible to scan the whole array in a standard microarray scanner (for example from Agilent Technologies) for fluorescence detection. Scanning of fluorescently labeled cells in this manner takes only a few minutes and the output, i.e. the results from all wells, will be given simultaneously.

The microplate may comprise at least a bottom plate and a microgrid plate. The microplate may further comprise a semi-permeable top membrane for sealing of the wells after cell seeding in order to, among other things, prevent evaporation. The membrane is semi-permeable in order to allow a fluid, for example gas or liquid, to enter and leave the wells. Sealing of the wells with a top membrane helps to keep the very small volumes of growth medium inside the wells. Sealing of the wells also prevents the cells from leaving their wells and thus ensures reliable results regarding cell maintenance when performing the method.
Alternatively, a layer of mineral oil may be used to seal the wells.

In the microplate for use in the methods, the bottom plate can be made of glass or any other suitable material. Furthermore, the microgrid plate can be made of silicon, or any other suitable material. Also, the top membrane can be made of polydimethylsiloxane (PDMS) or any other suitable material.

The microplate may be formed from a sandwich structure with three levels: a bottom plate, an etched microgrid plate and a semi-permeable top membrane.

The microplate may furthermore have the format of a standard microtiter plate (size 128 x 86 mm), in order to facilitate implementation in clinical labs and standard instruments. The microplate can for example have an anodically bonded glass bottom. Alternatively, it can be clamped together with a selected surface to enable surface modifications (e.g. fibronectin, lysine, gelatin, cell seed layers, etc) of the whole bottom plate before assembly into a sandwich structure.

If desired, the microplate may be washed and reused in the methods.

A microplate comprising a flat glass bottom of variable thickness allows all kinds of imaging analysis. Many interesting cellular features can be studied with, for example, confocal microscopy using different labeling techniques and/or different labels for different cells. Cells may for example be fluorescently labeled. Fluorescence labeling can be obtained for example by addition of fluorescently labeled affinity compounds, live/dead cell stain (e.g. Calcein AM), nuclear dye (DAPI), organelle specific trackers, etc. Furthermore, the presence of a transparent semi-permeable membrane enables optical detection from both top and bottom of the plate, i.e. both upright and inverted microscopy is possible.

The methods enable seeding and cultivation of all cell types, comprising adherent and non-adherent cells. Non-adherent cells, for example, have in particular been difficult to order into single cell arrays due to the difficulty of tethering artifacts. Tethering artifacts for both adherent and non-adherent cells may however be avoided. The wells of the microplate used when performing the methods provide suitable compartments for all cell types, comprising adherent and/or non-adherent cells. The wells can further be sealed with a semi-permeable top membrane. Accordingly, the problem of disruptive anchoring of cells will not arise in the methods.

The methods of seeding and cultivation can be used for any primary cells or cell lines. As non-limiting examples, the methods may be used for neoplastic cells, such as for example human cancer cells, and for stem cells. Successful seeding and cultivation of human leukemia cells, human myeloma cells, human and mouse embryonic stem cells and adult neural cells according to the disclosed methods are presented in the appended Example 1. Seeding and cultivation of cancer cells, for example, enable rapid and controlled clinical testing.

When used for seeding and cultivation of stem cells, the methods furthermore enable identification of stem cells within a cell population, monitoring of cell differentiation, screening of markers on single stem cells/clones, etc.

In the method of cultivation, the analysis comprises analysis of cell response to the analytic medium. The cell response may for example be selected from cell viability, cell death, clone formation and cell resistance.

Simultaneous analysis of several thousands of individual cells per well is enabled by the methods disclosed. By controlled seeding of individual cells to predefined locations of the microplate, analysis of single cell heterogeneity and colony formation, for example in relation to drug sensitivity, can easily be accomplished.

Large cell samples comprising millions of cells are however not a requirement for performing the methods. On the other hand, both large and small cell samples can be used with the methods. Compared to previously known methods, the methods can be performed using a lower number of cells. The methods are therefore well suited for, for example, screening of limited amounts of patient or primary cell samples. The methods enable investigators and scientists to analyze characteristics of patient samples on a single cell level, potentially leading to a more optimized treatment. As shown in the appended example, cell consumption drastically decreases to 0.6 % of the cells needed in conventional methods (Larsson, et al, Int J Cancer 50, 177-185 (1992)). Conventional methods often require cell samples comprising millions of cells, since analysis of for example cell sensitivity to drug addition is measured as an average of cell response for 50 000-100 000 cells.

In some embodiments of the methods, at least two cells are seeded into at least one well of the microplate. By seeding two or more cells into one well of a microplate, or alternatively by seeding single cells, cell-to-cell interaction studies can be performed in a high-throughput manner. Another possibility is seeding of different types of cells into one well. Thus, controlled mixtures of different types of cells in different wells can be obtained.

The methods can be used in diagnostics, research, drug discovery, agriculture, stem cell analysis, etc. In addition, the methods are suitable for use in clinical medicine, for identification of potential drug targets, optimization of a lead compound for a specific disease, test of cell viability, test of toxicity when cells are exposed to a specific compound, test of sensitivity to a specific compound, basic biological research, etc. The term compound refers to any chemical compound.

Moreover, the methods can be used for studying cell response to drug gradients, cell morphology, temperature gradients, as well as electrical stimulation of the cells. The methods could also be one step towards individualized medicine and/or pharmacotherapy in order to achieve more accurate treatment of a patient. Preclinical testing of drugs by the methods could reduce the need of animal testing.

The methods allow for similar detection and analysis techniques in a microplate as can be accomplished in any other microplate. However, the methods enable detection and analysis for thousands of cells in parallel. In addition, the methods enable real-time monitoring of changes over time of protein localization and metabolite contents, which can be done for thousands of single cells in parallel. The methods could be used for PCR in-plate, cell lysis, RNA/DNA analysis, protein detection, stem cell research and differentiation studies, etc.

The methods for high-throughput seeding and cultivation of single cells followed by single cell analysis have the potential to become important diagnostic tools when studying tumor cells from for example leukemia patients, with respect to e.g. heterogeneity, proliferation, apoptosis and sensitivity to chemotherapeutics at the single cell/clone level.

As described above, methods are provided to allow for rapid cell seeding into individual wells that can be studied over time. The cells can be studied with respect to morphology, clone formation and viability based on microscopy detection. The method may also allow for genetic analysis of cell material held in microwell(s) of a microplate.

In the method of genetic analysis, as defined in the claims, single cells, or clones expanded from single cells, are contained in individual wells of a microplate comprising at least 5 wells/cm². The single cells, or clones from single cells, are lysed and nucleic acids may thus be obtained in the individual wells. The nucleic acids may subsequently be amplified by PCR to provide amplified PCR product in the individual wells. Analysis of amplified PCR product may further allow mutations to be detected independently of the frequency of normal cells whereby single cell information may be linked to information from genetic analysis. The method may accordingly enable mutation analysis of clonally expanded single cells, as well as investigations and comparisons of cell culture heterogeneity, e.g. the proliferation tendencies of individual cells, and genetic heterogeneity, e.g. mutation frequency in tissues of interest.

Use of clonally expanded cells for DNA amplification may provide an increased amount of starting material as compared to DNA amplification from single cells. DNA amplification from clonally expanded cells may thus be considered as more robust than DNA amplification from single cells.

In one embodiment, the method of genetic analysis may comprise seeding of at least one cell sample into at least one well of said microplate. Cell seeding may for example be accomplished by using a flow cytometer apparatus, for example a flow cytometer comprising sorting means. One example of a flow cytometry apparatus which may be used is a fluorescence activated cell sorter (FACS).

After cell seeding, the microplate may be incubated under cell cultivating conditions. Cell cultivation for a predetermined period of time enables expansion of cell clones from the single cells contained in the individual wells. Clones of varying size may thus be generated in the wells where seeding was performed. During cell cultivation, or after cell cultivation, biological properties of the cells or cell clones contained in the individual wells may be analyzed. Results from such analysis may further be linked to, or compared with, results from analysis of genetic material.

As shown in the appended Examples, the method of genetic analysis may enable cell cultivation, cell lysis, PCR and analysis of PCR product in individual wells of a microplate. A low number of adherent cells may be subjected to PCR directly in the microwells in which they were cultured. Alternatively, solid-phase PCR may be run on e.g. magnetic beads and PCR products may be detected directly after the reaction. If the wells of the microplate are sealed, detection may thus be performed without removing the sealer.

PCR products may further be provided with capture labels such as any biological or chemical label, such as for example antibodies and fragments of antibodies, biotin, affinity reagents, DNA binding molecules and molecules derived from Protein A.

In the method, capture means are employed to capture amplified PCR product, for instance via said capture label. Examples of capture means are streptavidin, antigens and antigen fragments, which for example may be provided on a surface in a microplate or on a magnetic bead. Other examples of capture means are any biological, mechanical or chemical means for capture. Capture means could for example be a filter positioned in a well in a microplate.

In one example of the method of genetic analysis, liquid handling is automated. Liquids may be dispensed manually or by using a (automatic) controlled liquid handling system, such as a conventional nano-dispensing system. A controlled liquid handling system may allow contamination-sensitive experiments by further minimizing inter-well contamination. In one example of the method of genetic analysis, a microfluidic system is integrated with the microplate.

In embodiments thereof, the method further provides denaturation of PCR product and genetic analysis such as minisequencing in-plate. Minisequencing was first described by Syvänen, et al in Genomics 8, 684-92 (1990), and as the name reveals it determines only a few nucleotides of a DNA sequence. After some modification, the technique now uses dideoxynucleotides (ddNTPs) as in the Sanger sequencing method to terminate elongation. In minisequencing only ddNTPs and no deoxynucleotides (dNTPs) are added, allowing only a single base to be extended, implying that the primers should be designed so that the SNP is the first base downstream of the primer. The ddNTPs can be labeled with different fluorescent dyes enabling many SNPs to be determined at the same time in an array format.

Following genetic analysis such as minisequencing, the method may further allow the genotype of cells held in individual wells to be studied and detected, e.g. in a high-throughput manner in an array scanner. Detection of PCR products in the microwells may for example be accomplished by employing one biotinylated and one fluorescently labeled PCR primer. In embodiments of the method, PCR products may be detected by allele-specific PCR, where each allele-specific primer is labeled with a specific reporter fluorophor.

The methods according to the invention will now be described in a non-limiting manner by the following Figures and Examples.

### Brief description of the appended figures

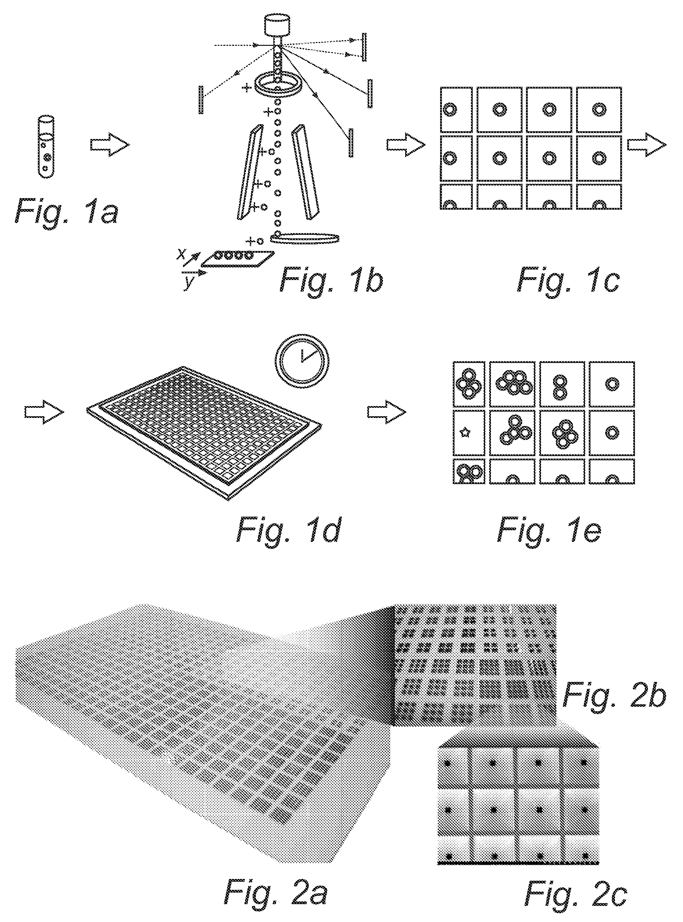

Figure 1 is a drawing that illustrates an example of a method of cell cultivation. Single cells from patient cell samples (a) are seeded by flow cytometry (b) into the microplate (c). The whole microplate is incubated (d). Analysis can be done instantly (c), during incubation (d) and/or after incubation (e).
Figure 2 is a drawing that illustrates a microplate useful in the methods of seeding and cultivation.
   a) displays a microplate having four sections with different well sizes. A 128 mm x 86 mm microplate having any of the displayed well sizes would contain 1536-6144 wells. Scale bar 1.5 cm.
   b) is an enlarged section of a) displaying the microplate with different well sizes.
   c) is an enlarged section of b) displaying the microplate with wells having tilted walls for facilitated cell seeding.

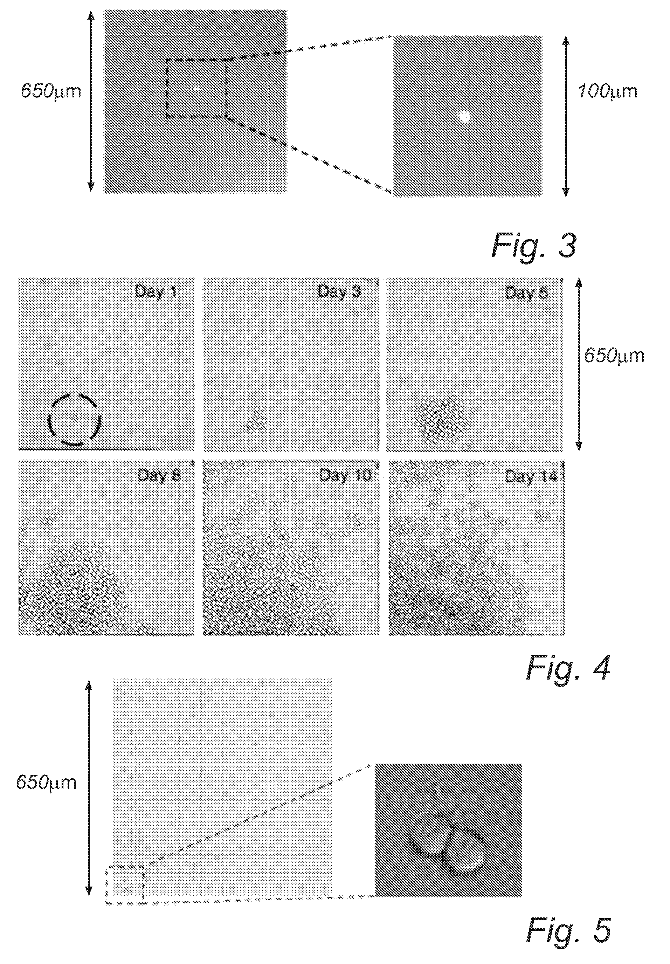

Figure 3 is a photo displaying a fluorescently labeled single cell in one well directly after cell seeding.
Figure 4 is a series of photos displaying clone formation in one well (day 1-14) starting with a single cell on day 1.
Figure 5 is a series of photos displaying proliferation of leukemia cell samples from a patient in one well. The enlarged section shows two daughter cells after 4 days of cultivation.

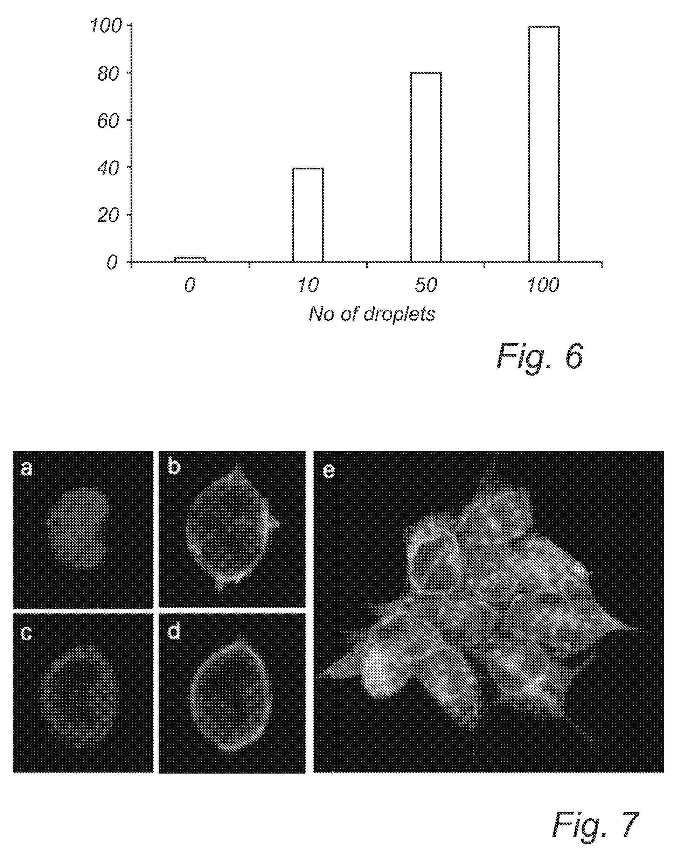

Figure 6 is a diagram showing percentage of dead cells after addition of lysis buffer (y-axis). The x-axis represents amount of lysis buffer in terms of the number of droplets added.
Figure 7 is a series of photos displaying confocal images (a-e) of cell proliferation for a single mouse embryonic stem cell (ES). Four antibodies were used for labeling of one single ES cell in one well after 1 (a-d) and 3 days of cultivation. Scale bar 10 µm.
   a) displays labeling of DNA (corresponds to the grey portion of the image).
   b) displays labeling of filamin (HPA ab, grey portion).
   c) displays labeling of calreticulin (grey portion).
   d) displays labeling of tubulin (grey portion).
   e) displays an overlay image of an antibody-labeled cell colony according to a-d) after 3 days of single cell cultivation.

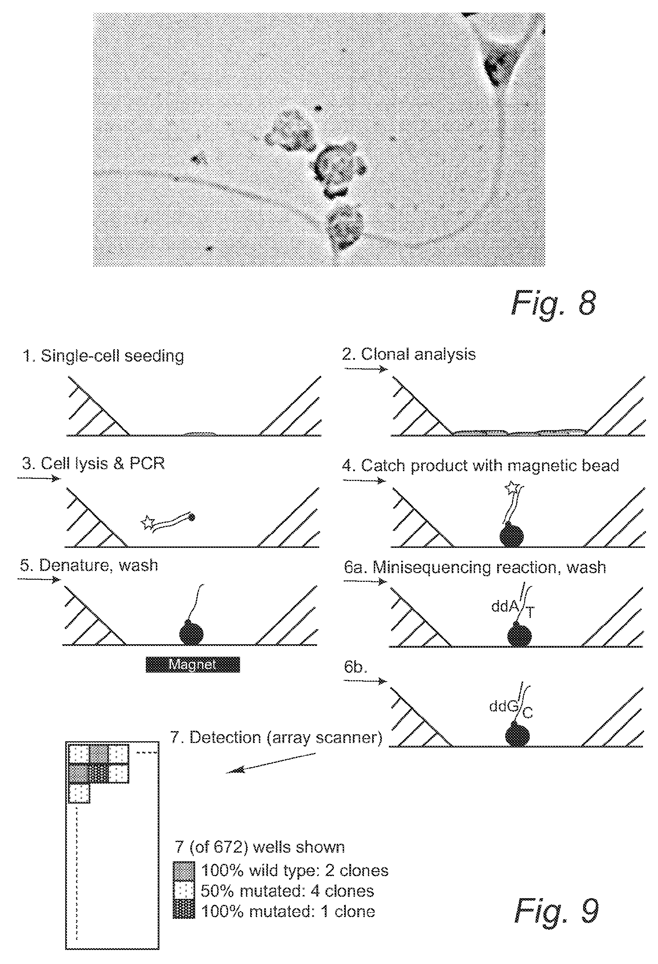

Figure 8 is a photo displaying a differentiated adult neural stem cell in a well after 3 days of cultivation (upper right corner).
Figure 9 is an overview of the method of genetic analysis of single cell samples. Following cell seeding and cultivation, the microplate is rinsed to remove growth medium leaving the adherent cells at the glass bottom of the microwells (1-2). PCR mixture is added, wells are sealed with a plastic film and the cells are lysed in a standard PCR thermocycler resulting in target DNA amplification (3). A primer pair with one biotinylated and one fluorescently labeled primer, allows capture of the fluorescent product to streptavidin coated magnetic beads and thereby detection of the product (4). To further investigate the genotype of the cells, the captured product can be denatured (5) and a single base mutation can be detected (6) by single base extension. Fluorescence microscopy can then be used for (high resolution) detection in some chosen wells. For screening of all wells simultaneously, an array scanner instrument would be the first choice (7).

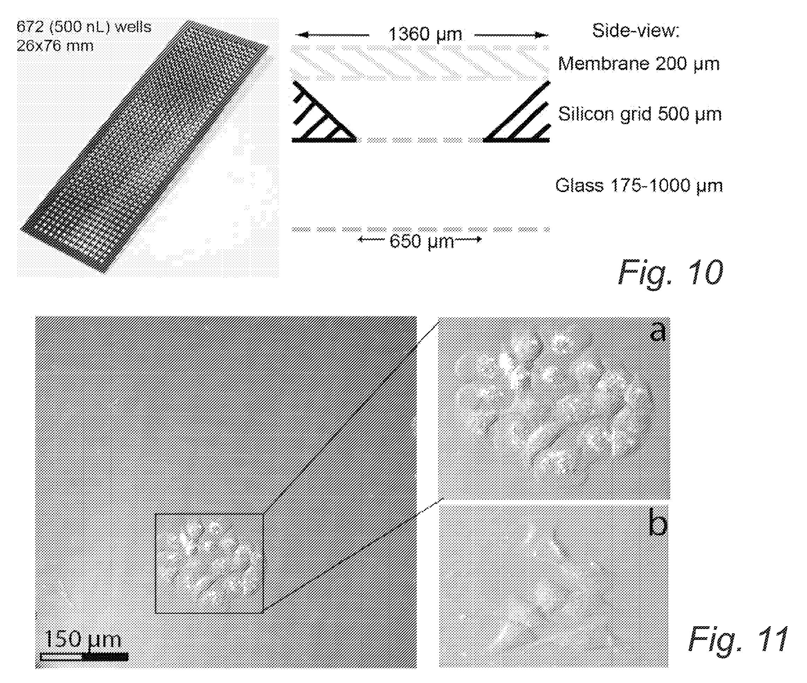

Figure 10 is a series of photos displaying the design of a microplate useful in the methods disclosed. The photo to the left displays a microplate consisting of 672 microwells, each with a 500 nl volume, enclosed in glass with the same dimensions as a standard microscopic glass slide. The photo to the right is a schematic side view of the microplate design. A glass bottom and a silicon grid are anodically bonded and a top membrane may be reversibly added for cell culturing.
Figure 11 is a series of photos displaying a) one single adherent A-431 cell and b) one single U-2 OS cell grown in microwells for three days, forming single clones. Micrographs were obtained using a 20x objective and DIC settings.

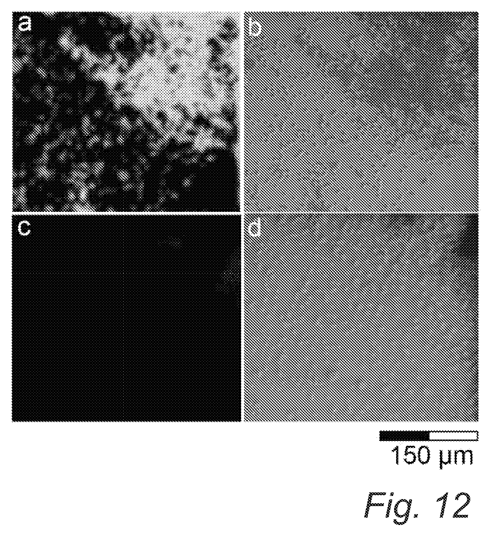

Figure 12 is a series of photos showing fluorescence detection of ds DNA bound to streptavidin-coated magnetic beads. FITC-fluorescence was detected using a fluorescence microscope (exc 488 nm), a 20x objective, and a longpass 505 filter (a,c). The beads were visualized in the bright field channel (b,d). The micrographs show the beads with captured PCR products a) before denaturation and c) after denaturation with 0.1 M NaOH, which has washed the fluorescent complimentary strands away.

### Examples

### Example 1: Seeding and cultivation of leukemic K-562 cells, leukemic patient cells and human myeloma cells

### Materials and equipment

### The microplate

The microplate used in all experiments below had a sandwich structure of three levels: a bottom glass plate, an etched silicon microgrid plate and a semi-permeable top membrane.

The microplate was produced with standard micro fabrication techniques involving nitride deposition, lithography, KOH silicon etching and nitride strip. The 500 µm thick silicon grid was anodically bonded to a 500 µm glass slide where after the plate was sawed into microtiter plate format (128 mm x 86 mm). The wells of the microplate had tilted walls (54.7°) to facilitate cell seeding. The well bottom was 650x650 µm and the top opening was 1360x1360 µm. The total plate thickness was 1 mm and the centre-to-centre distance between wells was 1500 µm. The plate had 3243 wells of which 256 wells were used. The well volume was 500 nl.

### Semi-permeable top membrane

Different semi-permeable membranes were evaluated for sealing of the wells, in order to optimize cell cultivation conditions and to prevent evaporation.

In total 9 membranes, sealing tapes or films were tested (table 1).

In general, evaporation tests and/or cell viability tests were performed for the different membranes. For the evaporation tests, cells were not necessarily seeded. For the cell viability tests, K562 cells were used. Growth medium and cells were simultaneously added to the wells of a microplate either manually by using a pipette or automatically by using a FACS. Thereafter, the wells were covered with the membrane and incubated in a cell incubator at 37°C, 5% CO₂ and elevated humidity. Cell clone formation was observed by light microscopy and cell viability was investigated by Trypan Blue staining.

Initially, 5 liquid-permeable hydrophilic membranes (1-5) were selected based on the idea that cultivation media could be exchanged during long cell cultivation periods. By having the cells in the bottom of the wells, and letting growth/cultivation media pass the filter membrane (permeable also to oxygen and carbon dioxide, necessary for cell cultivation), it was believed that cells could stay alive longer. After covering the wells with the membranes, the membranes were wetted by dropwise addition of a small amount of growth medium onto the membrane. Membranes 1-5 did not seal optimally onto the plate. In addition, the membranes dried up whereby cell cultivation was made impossible.

Therefore, 3 different gas-permeable (but not liquid permeable) membranes, or sealing films, were chosen for testing (6-8). These membranes were randomly chosen for their known suitability for cell cultivation in standard microtiter plates. However, for membrane no. 6, only 50 % of the cells were alive after 45 h. Cell cultivation with membranes 7-8 resulted in 100 % dead cells after 45 h.

Thus, the commercially available plastic membranes or films did not result in sufficient sealing on the silicon surface of the microgrid plate. Also, utilization of these membranes or films resulted in bubble formation in the wells and, above all, unsatisfactory cell viability.

Finally, a home-made membrane made out of PDMS was tested. The membrane was casted by mixing Sylgard® 184 Silicone Elastomer according to the manufacturer's instructions (Dow Corning). Mixing was performed manually with a pencil-like stirrer. The elastomer mix (total volume 10 ml) was then poured into a plastic lid (facing top-down, normally used for multi well plates, from Greiner bio-one). The thickness of the membrane was approximately 2-3 mm and the dimensions matched those of a standard microtiter plate. The plastic lid containing the elastomer mix was cured in 70°C for 2 h. After a membrane had formed, it was peeled off the plastic lid, wrapped in aluminum foil and autoclaved (121°C for 20 min). The membrane was kept sterile in the aluminum foil until being manually placed on top of the microplate. The resulting semi-permeable membrane was transparent. Before covering the microplate with the membrane, a few drops of growth culture media were placed on the microplate to create an excess of liquid and to thereby prevent air bubble formation within the wells.

A cell suspension was added manually to the microwells, whereby the plate was sealed with membrane no. 9. The plate was incubated in a cell incubator and after 72 h the viability was determined with Trypan Blue (dead cell stain). The membrane was removed, followed by addition of Trypan Blue to the microwells. After 5 minutes the dead cells stained blue, whereas the living cells stayed unstained. Detection was done by manually screening the plate, well by well, in a light microscope. The viability was very high, with only a few percent of dead cells. It was concluded that cell viability in the microplate was as high as in standard (petri dish) cell cultivation methods when compared to cell cultivation in a control petri dish. Cells had splitted into colonies (i.e. formed clones) which were observed with light microscopy in the same manner as described above.

In cross-comparative studies between the sandwich-structured microplate and other types of plates, i.e. conventional 24- and 384-well plates, and membranes 6 and 9, it was shown that both membrane types resulted in good cell viability for the conventional plates, whereas for the sandwich-structured microplate, membrane no. 6 gave low cell viability and membrane no. 9 gave very high cell viability. Cell viability was measured after 72 hours.

The same membranes were also compared in a viability study wherein single cell seeding was performed with a FACS. Again, in wells covered by membrane no. 6 no clone formation had taken place, whereas in wells covered by membrane no. 9 clone formation had taken place and differentiation was observed until 12 days after seeding.

Thus, a PDMS membrane was used in the following experiments.

**Table 1.**

| | **Membrane material** | **Supplier** | **Catalogue no.** | **Pore size (µm)** |
|---|---|---|---|---|
| **1** | PVDF, hydrophilic | Millipore | HVLP14250 | 0.45 |
| **2** | PVDF, hydrophilic | Millipore | GVWP14250 | 0.22 |
| **3** | Polyethersulphone, hydrophilic | Millipore | GPWP14250 | 0.22 |
| **4** | Mixed cellulose esthers, hydrophilic | Millipore | GSWP14250 | 0.22 |
| **5** | Mixed cellulose esthers, hydrophilic | Millipore | HAWP14250 | 0.45 |
| **6** | BreathEasy, hydrophobic | Diversified Biotech | BEM-1 | |
| **7** | BreathEasier, hydrophobic | Diversified Biotech | BERM-2000 | |
| **8** | Area Seal, hydrophobic | Web Scientific | WTS-7014 | |
| **9** | Polydimethylsiloxane | In-house | | |

A way of further preventing evaporation during long cell seeding procedures would be to pause the seeding and cover the part of the microplate that has already been seeded with cells with a semi-permeable membrane, before continuing seeding the remaining parts of the plate.

### Apparatus for cell seeding

The first step towards long-term single cell analysis is to place a single cell into each well of the microplate, in a highly controlled and high-throughput manner. A FACS (FACSVantage SE Cell Sorter (BD Biosciences)) was used for performing the cell seeding. Figure 3 demonstrates the precision of cell seeding using a FACS.

### Cell seeding

The clean plates and semi-permeable top membranes were autoclaved for 20 min at 121°C to obtain a sterile cell culture environment. An excess of recommended growth medium was added to the plate, and manually distributed evenly to all wells by an autoclaved hand scraper (VWR) or sterile cell scrapers (Falcon, for one-time use only). Then, automatic single cell seeding into predefined wells of the microplate was carried out using the FACS. A motorized x/y-stage combined with the software CloneCyt Plus (BD Biosciences) was used to control the exact position of every cell sorted in the plate. Selection of the cells to be seeded was performed based on their individual FSC/SSC (forward scatter/side scatter) properties (i.e. size and granularity). The cells were analyzed at a rate of approximately 100 cells/s. A representative set of cells was sought, meaning that all viable cells in the sample were selected for seeding into the plate. The cells were sorted directly from recommended growth medium in concentrations ranging from 10⁵-10⁶ cells/ml. FACS Clean solution (BD Biosciences) was let through the fluidics of the FACS prior to experiments to obtain sterility. The x/y-stage and the surrounding of the fluidics outlet was cleaned with 70% EtOH. During analysis, sterile 0.25x PBS was used as sheath buffer. After cell seeding, a semi-permeable membrane was applied to the top of the microplate to seal the wells. Random cell seeding by manual pipetting can be utilized, either by addressing individual wells or by addressing all wells simultaneously. In the latter, a volume of 800 µl of cell suspension is dispensed onto the chip, spread over the chip area and the cells are randomly settled in the wells by adding a cell culture top membrane. The plate, loaded with single cells, was incubated in a humidified atmosphere at 5% CO₂ and 37°C for 3-14 days.

The time needed for seeding single cells was one second per well. The resulting well occupancy of single cells was approximately 97%, i.e. in approximately 97% of the wells, single cells had been seeded as intended. Fluorescence analysis was used for estimation of well occupancy.

### Leukemia cells

Single leukemic K-562 cells (ECACC No. 89121407) were seeded as described above and cultured in the microplate in RPMI-1640 + L-glut, supplemented with 10% Foetal Bovine Serum (GIBCO) and antibiotics/antimitotics in a humidified atmosphere, 5% CO₂, 37°C up to 14 days. The humidity chambers consisted of home-made plate holders in plastic trays (Bio-Rad), where the bottom was covered with deionized water.

The K-562 cell line was cultured in parallel in standard petri dishes (BD Falcon), throughout the whole study.

Less than 5% of the wells per microplate were excluded from the study due to air bubble formation during sealing, which generally make cell detection impossible. The exclusion number was determined by visual inspection (if needed in a microscope) of fluorescently labeled K-562 cells for 256 neighboring wells in 3 replicates followed by statistical calculation.

### Microscopy analysis

The K-562 cells were fluorescently labeled with Calcein AM (Molecular Probes) according to the manufacturer's instructions. May-Grunewald/Giemsa (MGG) staining (VWR) of cells enabled observation of different types of cells. For viability tests, the Trypan exclusion method was used (Sigma). Cell proliferation observations were performed with an Olympus BX51 light microscope, outfitted with a manually adjustable x/y-stage and a digital camera (Olympus Camedia C-4000 zoom). Using a 10x objective lens, pictures were taken at various time points. Fluorescence imaging for studying cell seeding accuracy was performed with a Zeiss LSM 510 Meta confocal microscope equipped with motorized x/y-stage.

### Leukemia cell samples from patients

Patient cell samples were purified with Ficoll-Paque (GE Healthcare) at Uppsala Academic Hospital (Uppsala, Sweden) and delivered in frozen aliquots of 10⁵ cells/ml. The mononuclear cells were thawed, centrifuged and suspended in a recommended growth medium, followed by immediate seeding using FACS into the plate. The same FACS sorting protocol, including preparations and incubations, was used for all cell types. The leukemia cell samples from patients showed the ability to grow in-plate (Figure 5).

### Proliferation analysis

The next step, following single cell seeding, was clone formation analysis and study of proliferation in the plate. K-562 leukemia cells were successfully seeded and cultivated in the plate for up to 14 days (Figure 4), which was followed by evaluation of viability and colony formation.

Cell viability in the microplate was comparable with viability in other cell cultivation systems. Single cell cultivation experiments in fifteen randomly chosen wells were terminated after 6 days, and cells were stained with the dead-cell dye Trypan Blue. This revealed an average of 95% viable cells in each well.

After two weeks of cultivation, up to 14 generations of cells were formed, which means that the initial single cells gave rise to more than 10 000 cells in each microwell. In addition, 256 neighboring wells were followed during 6 days, which showed that 93% of all single K-562 cells gave rise to colony formation. The colony size differs, as expected, from well to well due to cell heterogeneity and non-synchronized cell line.

May-Grünewald/Giemsa (MGG) was used for staining in-plate. MGG is a stain for air-dry cytology preparations, commonly used for distinction between different blood cells. First, the membrane was peeled off the microplate, while still keeping the cells in their respective wells. The growth medium was evaporated to dry the cells in-plate. In some cases the whole plate was stored at -18°C for later analysis, after drying of the cells. The plate was maintained in room temperature for a couple of minutes before staining. No differences in results between freezed and non-freezed samples in-plate were detected, which implies that cells can be analyzed directly (fresh) or thawed for this type of staining.

Furthermore, cell consumption was only 0.6% of the number of cells needed in conventional methods, see Larsson et al, Int J Cancer (1992) (*supra*).

### Human myeloma cells

Other cell lines that were cultivated in-plate were the human myeloma cell line RPMI8226 and the doxorubicin-resistant counterpart RPMI8226_{Dox40}. These cell lines have some adherent-like properties and tend to sit on the bottom of the well while proliferating.

The human myeloma cell lines RPMI8226 and RPMI8226_{Dox40} (ECACC No 87012702 and primary leukemic cells) were cultured in parallel in flasks (Nunc) in the same growth medium as the leukemia cells above (RPMI-1640 + L-glut, supplemented with 10% Foetal Bovine Serum (GIBCO) and antibiotics/antimyotics in a humidified atmosphere, 5% CO₂,). 0.24 µg doxorubicin was added per ml RPMI8226_{Dox40} cell culture once a month to maintain the doxorubicin resistance of this cell line. The RPMI8226_{Dox40} cell line was trypsinized (GIBCO) before passages to remove cells tending to adhere from the surface of the culture flask.

### Addition of drugs

The FACS was used for addition of lysis buffer (0.1 % Triton X-100) as a model drug substance in a proof-of-concept study. Drug addition, i.e. here, the addition of lysis buffer, was performed in the same manner as the cell seeding. Thus, a portion (droplet) of lysis buffer was added to chosen wells in a highly controlled manner, see Figure 6. It was shown that the percentage of dead cells (y-axis) increased with an increased amount of lysis buffer (x-axis).

Another option for investigating cell response to drug addition is to seed several cell types and treat them similarly. This was done with the chemotherapeutic drug doxorubicin, and the results are shown in Table 2. Five microplates were seeded with two cell types: doxorubicin sensitive myeloma RPMI8226 cells and its doxorubicin resistant sub line RPMI8226_{Dox40}. Growth medium spiked with five different concentrations of doxorubicin was manually added to respective plate before cell seeding. Results after 6 days of incubation clearly showed that only resistant cells can proliferate at higher drug concentrations, demonstrating the capability of this single cell analysis methodology as a tool for drug resistance screening. Table 2.

| **Doxorubicin [µM]** | **Sensitive (RPMI8226)** | **Resistant (RPMI8226_{Dox40})** |
|---|---|---|
| 10 | - | - |
| 0.2 | - | Cell growth |
| 0.02 | - | Cell growth |
| 0.002 | Cell growth | Cell growth |
| 0.000 | Cell growth | Cell growth |

### Screening for stem cell/progenitor markers on single cells

High throughput coating procedures for improved cultivation conditions were developed for mouse embryonic stem (ES) cells and adult (primary) neural stem (NS) cells (Karolinska Institute, Stockholm, Sweden) as well as the three adherent cell lines U-2 OS (osteosarcoma), SH-SY5Y (neuroblastoma) and SK-BR-3 (adenocarcinoma), wherein the three latter were used as controls for adherent conditions. Single ES cells and NS cells were cultivated for 1-3 days. ES cells were also cultivated for one week.

For improved adherent cell culturing conditions, coating procedures for the whole plate were optimized (table 3) for mouse ES cells and mouse NS cells as well as the adherent human cell lines. The same coating protocol was used for the cell types and coatings according to table 3. Therefore, only coating of plates for cultivation of ES cells is described. Microplates of array format (76 mm x 24 mm), each comprising 1081 wells, were used. The plate was pre-coated with 800 µl 0,2 % gelatin solution. The gelatin solution was added to the plate and distributed evenly over the plate with a cell scraper (BD Falcon). The plate was left in room-temperature for 5-15 minutes. Thereafter the plate was rinsed with 3x5 ml sterile 1 x PBS (phosphate buffer saline) by gently pipetting PBS over the entire plate. Lastly, the plate was quickly turned upside down on a tissue, to remove liquid from the wells. Cell viability in the coated plate was thereafter investigated by seeding and cultivating ES cells (in recommended growth medium). Here seeding was manually performed by pipetting cells into wells.

Poly-L-lysine (0.1 mg/ml) and fibronectin (10 µg/ml) were found to provide the best conditions for cell proliferation for NS cells and adherent cells respectively.

After establishing optimal coating conditions, single cell seeding and cultivation were performed in pre-coated plates. For ES cells, two plates of array format were coated according to the protocol above. Growth medium (recommended growth medium for ES cells) was then added to the wells of the plates. The same seeding protocol as for leukemia cells was used. One plate was incubated for 1 day (fig 7a-d) and the other plate was incubated for 3 days (fig 7 e).

Protocols for fixation, antibody labeling and fluorescence imaging were developed for single stem cells/clones in-plate.

The plates were gently (not to remove adherent cells) washed with PBS, by either dipping the plates into a beaker containing PBS or by pipetting 2x5 ml PBS over each of the plates. Thereafter, ice-cold MeOH was added and the plates were placed in the freezer. After 5 minutes, the plates were removed from the freezer and washed by dipping the plates into a beaker containing 1x PBS. The plates were quickly turned upside down on a tissue to remove liquid from the wells. Primary antibody in blocking solution (4% fetal bovine serum (FBS) in PBS) was added. The plates were left overnight in 4°C.

The following day the plates were washed 3x5 minutes with 1 x PBS in a beaker. Secondary (light sensitive) conjugated antibodies Alexa 488, Alexa 555 and Alexa 647 in blocking solution (4% FBS in PBS) were added and the plates were left for 1.5 hours in room temperature. 1 µl secondary antibody (antibody concentration 2 mg/ml) was added to 1 ml blocking solution. The plates were washed 2x3 minutes with PBS (in a plastic beaker). 0.300 µM nucleus-dye DAPI (1 µl DAPI in 1 ml PBS) was added and the plates were left for 4 minutes in room temperature. The plates were washed 3x5 minutes with PBS (in a plastic beaker), sealed with a membrane and put in the fridge.

Single ES cells were cultivated for from 1 to 3 days and showed good results on single cell proliferation. When clones had been formed, the fixation and antibody labeling protocol as described above was performed in-plate followed by fluorescence imaging (Figure 7a-e).

Adult NS cells were also cultivated (in recommended growth medium) in a microplate of array format with the aims of a) screening single stem cells in uncoated wells to find undifferentiated stem cells (neurosphere forming cells), and b) plating stem cells in coated wells and study cell differentiation (Figure 8). Subsequently, NS cells were fixed and stained in-plate for a glial specific marker after 3 days of cultivation (not shown). In addition, weeklong ES cell cultivation was performed. For weeklong ES cell cultivation (not shown), a method for growth medium renewal was established. Thus, the membrane was removed and the plate was rinsed with new growth medium for facilitating long-term cultivation of undifferentiated ES cells.

**Table 3.**

| **Cell type** | **Coating** | | |
|---|---|---|---|
| | **Poly-L-lysine mg/ml)** | **(0.1 Fibronectin (10 ug/ml**) | **Gelatin (0.2 % in PBS)** |
| Embryonic Stem Cell | | | X |
| Adult Neural Stem Cell | X | | |
| U-2OS | | X | |
| SK-BR-3 | | X | |
| SH-SY5Y | | X | |

### Example 2: PCR amplification and genetic analysis in a microplate

In an aspect of the invention, genetic analysis can be correlated to cell information, as exemplified in the following. Here the inventive methodology is demonstrated by analyses of two cell human lines: one wild type and one with a single-base mutation in the p53 gene, cultured in a microplate.

### Materials and methods

### Microplate fabrication and design

Fabrication of the microplate has been previously described (Lindstrom et al, Electrophoresis 29, 1219-1227 (2008)). In the present study, microplates of standard slide format (76 × 26 mm²) with 672 wells were used. Each well had a bottom size of 650 × 650 µm² and a 1360 × 1360 µm² opening at the top, resulting in a volume of 500 nl per well (Figure 10). The wells were designed with sloped walls to facilitate cell seeding. The microplates were of sandwich-layout with a 500 µm silicon grid anodically bonded to a glass bottom plate of various thicknesses depending on the selected detection method (175 µm for high resolution imaging and up to 1000 µm for low resolution scanning). Transparency of the wells was provided for cell culturing by a transparent gas-permeable top membrane of polydimethylsiloxane (PDMS; Sylgard 184, DowCorning, Midland, MI) as described by Lindstrom *et al* (*supra*). The thickness of the membrane used here was however selected from approximately 200 µm thickness and approximately 300 µm thickness. The microplate and the top membrane were autoclaved before use.

Alternatively, microplates of 72 × 36 mm² with 1081 wells may be used.

### Cells and media

The human osteosarcoma cell line U-2 OS (ATCC-LGC Promchem, Teddington, UK) harbours a wild type p53 gene and the human epithelial carcinoma cell line A-431 (DSMZ German Collection of Microorganisms and Cell Cultures, Braunschweig, Germany) has a transition mutation in exon 8 of the p53 gene (G>A in codon 273, as described in Harlow, et al, Molecular and cellular biology 5, 1601-1610 (1985), and Kwok et al, Cancer research 54, 2834-2836 (1994), only one allele present, Reiss, et al, Oncology research 4, 349-357 (1992)). Both cell lines were cultured in McCoy's 5a medium with 1.5 mM L-glutamine (Lonza Biowhittaker, Basel, Switzerland) supplemented with 10 % fetal bovine serum and 1 % antibiotic/antimycotic solution (both from Invitrogen, Carlsbad, CA) in 5 % CO₂ at 37°C. The cell lines were maintained, during performance of the microplate experiments, in 100 mm cell culture dishes (BD Falcon, BD Biosciences, San Diego, CA) and split sub-confluence using a 0.25 % Trypsin/0.53 mM EDTA solution (Invitrogen).

### Cell culturing on microplate

For enhanced cell adhesion on glass, the microplate was pre-coated with 12.5 µg/ml human fibronectin (BD Biosciences) for 30 min at room temperature in a laminar air-flow bench, by covering all microwells with a total of 800 µl fibronectin. Excess fibronectin was rinsed out with sterile PBS, whereafter the wells were emptied using UV-treated tissues (Precision Wipes, Kimtech Science, Roswell State, GA) to absorb the remaining liquid. The cells were manually seeded onto the microplate at a concentration of 10-40 cells/µl. (approximately 5-20 cells/well) unless otherwise stated, resulting in approximately five cells per well. A top membrane was added to the microplate to seal the wells, prevent evaporation, and contamination, before incubation overnight at 37°C, in a humidified atmosphere with 5 % CO₂. For long-term cell culturing, here approximately one week, the top membrane was removed and spent medium in the microwells was replaced by rinsing the microplate with fresh culture medium in less than a minute.

### PCR amplification from human DNA template using test primers

Initially, the following set of test primers: forward (5'-TCAGATTGAGCATACTAAAAGTGACTC- 3') and reverse (5'-CAGCAAATGCTTGCTAGACC-3') (MWG-Biotech AG, Ebersberg, Germany), targeting exon 11 of the cystic fibrosis gene, were chosen to investigate whether PCR reactions could be successfully performed in the microplate. A PCR reaction mixture containing 1x PCR buffer (50 mM KCl, 2 mM MgCl₂, 10 mM Tris-HCl pH 8.5 and 0.1 % Tween 20), 0.2 mM of each dNTP (GE Healthcare, Uppsala, Sweden), 0.2 µM of each primer, 0.6 U/µl Taq DNA polymerase (produced in-house unless otherwise stated) and 0.6 ng/µl human template DNA (isolated from healthy individuals) was prepared. Aliquots of 50 µl of the PCR reaction mixture were placed in conventional plastic 0.2 ml PCR tubes (Applied Biosystems, Foster City, CA). 50 µl of the PCR reaction mixture was added to the microplate and the wells were sealed by covering the microplate with a plastic plate sealer (InVitro, Stockholm, Sweden). The reaction mixture was initially heated to 94°C for 5 min followed by 40 PCR cycles of 30 s at 94°C, 30 s at 55°C and 30 s at 72°C, then a final extension of 72°C for 10 min. All PCR amplifications were performed in a GeneAmp™ PCR system 9700 (Applied Biosystems) with the microplate and tube amplifications being run simultaneously in the same heating block.

### Cell lysis and PCR amplification of U-2 OS and A-431 cells

After initial verification of successful PCR amplifications in the microplate, experiments were performed with wild type (U-2 OS) and p53-mutated (A-431) cells.

Following cell culturing, cell lysis was performed in the microplate. The top membrane was removed, the microplate was rinsed with 20 ml sterile PBS followed by 10 ml sterile deionized H₂O and the wells were left to air-dry for approximately 1 h at room temperature (RT) in a laminar air-flow bench. The PCR reaction mixture (as described above) was added and the microplate was sealed with plastic plate sealer.

To lyse the cells in the control reactions in conventional plastic PCR tubes, the cells were incubated at 65°C for 10 min then cooled on ice. The cell lysate was then added to the PCR reaction mixture to a final concentration corresponding to the cell concentration after culturing in the microplate. This protocol for cell lysis also proved to work well for cell lysis in the microplate. The protocol comprising air-drying for one hour was however chosen for the microplate experiments, since no difference could be seen between the two protocols as regards the PCR.

Negative controls with neither template DNA nor cells were routinely run in parallel with the PCR reactions comprising cells. For PCR reactions using cells, the initial denaturation step at 94°C was prolonged to 10 min (rather than 5 min when the test DNA template was used). For amplification of exon 8 of the p53 gene, a primer pair previously used by Berg et al (Clinical chemistry 41, 1461-1466 (1995)) was used: a forward FITC (F) labelled primer (5'-F-ACAGGTAGGACCTGATTTCC-3') and a reverse biotinylated (B) primer (5'-B-TGAATCTGAGGCATAACTGC-3') (both from MWG-Biotech AG). For this primer pair, an annealing temperature of 53°C was employed. All other PCR reaction mixtures and cycling conditions were as described in the previous section, PCR amplification from human DNA template using test primers.

### Capture of PCR products

The biotinylated products (both in conventional plastic PCR tubes and in the microplate, after removing the plastic plate sealing from the microplate to allow evaporation of liquid) were captured on streptavidin-coated paramagnetic polystyrene beads (Dynal Dynabeads® MyOne™ Streptavidin C1, Invitrogen). The beads were washed three times in PBS followed by dilution in PBS. Washed beads (0.5 mg/ml) were then added to each well in a volume of 500 nl and incubated with the PCR products for 15 min at RT whereupon the microplate was rinsed with PBS while placing a magnet (LifeSep™ 96F, Dexter Magnetics, Hicksville, NY) beneath the microplate to maintain the beads in their respective wells while washing.

As a control, PCR products were captured in conventional plastic PCR tubes. To capture the PCR products from a 50 µl PCR reaction in a conventional PCR tube, an equal volume (50 µl) of washed beads (1 mg/ml) was used and the mixture was incubated for 15 min at RT before washing three times with 50 µl PBS.

Throughout the study, to ensure that the specific, expected products had been generated by PCR reactions in the microplate, products from a number of wells were collected to obtain a suitable volume using a 1 ml disposable plastic syringe (BD Plastipak™, BD Biosciences) equipped with a Ø 0.4 x 25 mm blunt end needle (B. Braun Melsungen AG, Melsungen, Germany), and subjected to gel electrophoresis and fluorescence detection.

### Denaturing and minisequencing

To analyze the sequences of the PCR products captured on beads, double-stranded DNA was denatured into single-stranded DNA, the beads and immobilized PCR products were incubated twice with 0.1 M NaOH for 5 min, washed three times with PBS and once with sterile H₂O while they were held in place by a magnet as described above. 500 nl of minisequencing reaction mixture containing 0.065 U/µl KlenThermase™ DNA polymerase, 1x KlenThermase™ buffer, 5 mM MgCl₂ (all provided by GeneCraft, Köln, Germany), 0.5 µM Cy5-ddATP, 0.5 µM Cy3-ddGTP (both from Perkin-Elmer, Waltham, MA) and 0.4 µM of 5'-ACGGAACAGCTTTGAGGTGC-3' primer (MWG Biotech AG) were then added to each microwell. The wells were subsequently sealed by covering the microplate with a plastic plate sealer, after which the reaction mixture was heated to 94°C for 5 s, followed by 15 min at 57°C, 5 min at 62°C, 5 min at 67°C, and 5 min at 70°C, then the microplate was kept in the dark at 4°C. After this, the microplate was again placed on the magnet, the plate sealer was removed, and washed as described above. The microplate was left to dry in the dark for 10 min, then the sequencing results were obtained by scanning the microplate in a microarray scanner. As a control, the reaction was performed in conventional plastic 0.2 ml PCR tubes and the resulting mixtures were dispensed into the microplate for detection.

### Fluorescence detection

To detect the fluorescently labeled PCR products captured on beads, a laser scanning confocal microscope (LSM 5 PASCAL, Carl Zeiss GmbH, Jena, Germany) with a 20x/0.5 Plan-Neofluar objective was used. FITC was excited at 488 nm and detected using an LP 505 filter. For detecting Cy3- and Cy5-labelled minisequencing products in all the wells simultaneously, a microarray scanner was utilized (Agilent Technologies, Santa Clara, CA).

### Results

### Cell culturing in microplate

In the present study, two adherent cell lines (epithelial carcinoma A-431 and osteosarcoma U-2 OS) were cultured in a microplate, starting with a single cell per well (Figure 11), or a few cells per well. In a preliminary experiment, cells of both lines were grown directly on the glass bottom in the microwells and in a second experiment the wells were coated with fibronectin before cell seeding. Cells proliferated in both cases, and no significant differences in cell growth parameters on the different surfaces were observed (data not shown). However, the fibronectin coating protocol promoted faster adherence and was therefore used in subsequent experiments.

A-431 cells are known to have a higher sub-cultivation ratio than U-2 OS cells and grew more rapidly both in the microplate and in conventional cell culture dishes. The difference in colony size between the lines can be observed in Figure 11.

### PCR amplification in microplate

PCR mixture was added to extracted DNA, spread on the microplate and subjected to thermo cycling. The amplification, using a standard PCR instrument, succeeded and a specific product was detected by gel electrophoresis. The PCR results were verified by pooling products from a number of wells (to obtain sufficiently large sample volumes) and analyzing them by gel electrophoresis followed by fluorescence detection. It was thus confirmed that PCR reactions can be performed in the microplate and that targeted sequences of cultured cells can be amplified in their respective microwells.

Then exon 8 of the p53 gene was amplified from A-431 and U-2 OS cells cultured in the microplate. Before running the PCR, cells were cultured overnight or for up to five days, resulting in a couple of cells or up to 100 cells per well, respectively. Longer cell-culturing times were not employed in this study, but due to the ease of changing culture medium in the microplate, the only limitation of long-term culturing is cell confluency, since each well can hold at most ca. 200 adherent cells. In initial control experiments, amplification from reaction mixtures with cell concentrations of 2 and 20 cells/µl in PCR reaction tubes succeeded, but as expected (since no enzymatic digestion was applied) at 200 cells/µl the reaction failed.

### Detection of PCR products

Streptavidin-coated magnetic beads was used to detect the PCR products in the microplate by employing one biotinylated and one fluorescently labelled primer at 488 nm using a fluorescence microscope (Figure 12a-b). Hence, it was possible to detect PCR products immobilized on beads in the microplate without removing uncaptured primer before detection, due to the locally high concentration of the fluorophore on beads. As shown in Figure 12c, the beads showed no auto-fluorescence with the settings used for excitation at 488 nm.

### Denaturation and minisequencing

Capture by magnetic streptavidin-coated beads allowed washing of PCR product in the microwells. Washing was further facilitated by the sloped walls of the wells. The PCR products captured on the magnetic beads were denatured in the microplate. Figure 12 shows the same well with (a-b) captured double-stranded DNA and (c-d) single-stranded DNA after denaturation with NaOH and washing away of the complementary fluorescently labeled strand.

Minisequencing was performed in the microplate from cell material amplified in conventional PCR tubes. The PCR products were captured and denatured in the microplate, then subjected to minisequencing, using Cy3-labelled ddGTP and Cy5-labelled ddATP. Hence, the minisequencing products of the wild type allele (G) in U-2 OS and the G>A mutated allele of A-431 were expected to yield Cy3 and Cy5 signals, respectively. However, stronger signals were observed in the red channel than in the green channel, and consequently a tendency for material from the wild type U-2 OS cell line to generate Cy5 background signals. This may have been due to poor quality of the Cy3-labelled ddGTP or perhaps to non-uniform incorporation of the labeled terminators by the polymerase employed. Furthermore, the beads showed a tendency to clump and stick to the corners and sloping walls of the microwells. However, in this study these phenomena did not affect the analysis of the cells' mutation status, and thus were not further investigated. The time required to detect the minisequencing products was less than 10 min since the signals in 672 wells were read simultaneously using an array scanner.

In conclusion, this study demonstrates the possibility of performing PCR amplification and DNA analysis of clonal, cultured cells in individual wells. By expanding single cells into clonal populations, lysing the daughter cells, amplifying the genetic material, performing a mutation analysis using minisequencing and detecting the products in all wells simultaneously using an array scanner, the described method provides a potentially valuable tool for mutation analysis screening. Alternatively, fluorescence microscopy and other detection methods could be employed (the footprint of the microplate is compatible with standard instrumentation and holders).

Thus, the method could be used for studying heterogeneity in single-cell proliferation and clonal assays in cancer- and stem-cell research.

## Claims

1. Method of genetic analysis of single cell samples comprising the steps of
a) providing at least one single cell sample contained in a microplate comprising at least 5 wells/cm², wherein each single cell sample is contained in an individual well of said microplate, said single cell sample being selected from a single cell and cells expanded from one single cell;
b) lysing each of said at least one single cell sample in each of said individual well(s) to obtain nucleic acids, such as DNA and RNA, from said at least one single cell sample;
c) amplifying a genetically relevant portion of said nucleic acids by performing a polymerase chain reaction (PCR) using at least one primer comprising at least one capture label to obtain a labeled amplified PCR-product in each of said individual well(s); and
d) analyzing said labeled amplified PCR-product in each of said individual well(s) wherein said analyzing comprises capturing said PCR-product in each of said individual wells via said capture label to a capture means in said wells.

2. Method according to claim 1, wherein said step a) comprises
(i) providing at least one single cell sample; and
(ii) seeding at least one cell of said single cell sample into at least one well of said microplate; and optionally
(iii) incubating said microplate under cell cultivating conditions.

3. Method according to claim 2, wherein step a) comprises
(iii) incubating said microplate under cell cultivating conditions; and
(iv) analyzing at least one relevant biological property in at least one well of the microplate.

4. Method according to claim 3, in which said analysis of step a) (iv) comprises analyzing a property selected from cell morphology, cell proliferation, clone formation capabilities, cell differentiation, cell-cell interactions and cell-division cycle.

5. Method according to any preceding claim, wherein said PCR of step c) is solid-phase PCR.

6. Method according to any preceding claim, wherein said capture means are magnetic beads.

7. Method according to any preceding claim, wherein step d) further comprises
(ii) denaturing and washing said captured PCR-product in said wells to provide a single-stranded DNA captured to said capture means;
(iii) genetically analyzing said single-stranded DNA in said wells.

8. Method according to claim 7, wherein said genetic analysis of step (iii) comprises detecting single nucleotide polymorphisms (SNPs) by performing single base extension on said single-stranded DNA in at least one of said wells.

9. Method according to any preceding claim, wherein said PCR of step c) is performed using at least one primer comprising at least one detection label.

10. Method according to any one of claims 1-6, wherein said analysis of step d) comprises
(i) detecting double-stranded PCR-products in at least one of said wells, optionally using a detection label.

11. Method according to claim 9 or 10, wherein said detection label is selected from the group consisting of fluorescent dyes.

12. Method according to any preceding claim, wherein said microplate comprises 5-700 000 wells/cm².

13. Method according to any one of claims 2-4, wherein said seeding is performed using a flow cytometry apparatus.

14. Method according to any preceding claim, wherein the cells are selected from neoplastic cells, human leukemia cells, human myeloma cells, and non-human stem cells.

15. Method according to any preceding claim, wherein said nucleic acid is selected from DNA and RNA.

## Patentansprüche

1. Verfahren zur genetischen Analyse von Einzelzellproben, umfassend die Schritte
a) Bereitstellen wenigstens einer in einer wenigstens 5 Vertiefungen pro cm² umfassenden Mikroplatte enthaltenen Einzelzellprobe, wobei die einzelnen Vertiefungen der Mikroplatte jeweils eine Einzelzellprobe enthalten, wobei die Einzelzellprobe aus einer Einzelzelle und von einer einzigen Zelle expandierten Zellen ausgewählt ist;
b) Lysieren jeder der wenigstens einen Einzelzellprobe in jeder der einzelnen Vertiefung(en), um Nukleinsäuren wie DNA und RNA aus der wenigstens einen Einzelzellprobe zu gewinnen;
c) Amplifizieren eines genetisch relevanten Anteils der Nukleinsäuren, indem eine Polymerasekettenreaktion (PCR) unter Verwendung wenigstens eines wenigstens eine Einfangmarkierung umfassenden Primers durchgeführt wird, so dass ein markiertes amplifiziertes PCR-Produkt in jeder der einzelnen Vertiefung(en) erhalten wird; und
d) Analysieren des markierten amplifizierten PCR-Produkts in jeder der einzelnen Vertiefung(en), wobei das Analysieren Einfangen des PCR-Produkts in jeder der einzelnen Vertiefungen über die Einfangmarkierung an ein Einfangmittel in den Vertiefungen umfasst.

2. Verfahren nach Anspruch 1, wobei der Schritt a) (i) Bereitstellen wenigstens einer Einzelzellprobe; und
(ii) Aussetzen wenigstens einer Zelle der Einzelzellprobe in wenigstens eine Vertiefung der Mikroplatte; und gegebenenfalls
(iii) Inkubieren der Mikroplatte unter Zellkultivierungsbedingungen
umfasst.

3. Verfahren nach Anspruch 2, wobei der Schritt a)
(iii) Inkubieren der Mikroplatte unter Zellkultivierungsbedingungen; und
(iv) Analysieren wenigstens einer relevanten biologischen Eigenschaft in wenigstens einer Vertiefung der Mikroplatte
umfasst.

4. Verfahren nach Anspruch 3, bei dem die Analyse unter Schritt a) (iv) Analysieren einer aus Zellmorphologie, Zellproliferation, Klonbildungsfähigkeiten, Zelldifferenzierung, Zell-Zell-Wechselwirkungen und Zellteilungszyklus ausgewählten Eigenschaft umfasst.

5. Verfahren nach einem vorhergehenden Anspruch, wobei es sich bei der PCR unter Schritt c) um Festphasen-PCR handelt.

6. Verfahren nach einem vorhergehenden Anspruch, wobei es sich bei dem Einfangmittel um Magnetkügelchen handelt.

7. Verfahren nach einem vorhergehenden Anspruch, wobei Schritt d) ferner
(ii) Denaturieren und Waschen des eingefangenen PCR-Produkts in den Vertiefungen, so dass eine an dem Einfangmittel gefangene Einzelstrang-DNA bereitgestellt wird;
(iii) genetisches Analysieren der Einzelstrang-DNA in den Vertiefungen
umfasst.

8. Verfahren nach Anspruch 7, wobei die genetische Analyse unter Schritt (iii) Nachweisen von Einzelnukleotidpolymorphismen (SNPs) mittels Durchführen von Einzelbasenverlängerung an der Einzelstrang-DNA in wenigstens einer der Vertiefungen umfasst.

9. Verfahren nach einem vorhergehenden Anspruch, wobei die PCR unter Schritt c) mit wenigstens einem wenigstens eine Nachweismarkierung umfassenden Primer durchgeführt wird.

10. Verfahren nach einem der Ansprüche 1-6, wobei die Analyse unter Schritt d)
(i) Nachweisen doppelsträngiger PCR-Produkte in wenigstens einer der Vertiefungen, gegebenenfalls unter Verwendung einer Nachweismarkierung,
umfasst.

11. Verfahren nach Anspruch 9 oder 10, wobei die Nachweismarkierung aus der aus Fluoreszenzfarbstoffen bestehenden Gruppe ausgewählt ist.

12. Verfahren nach einem vorhergehenden Anspruch, wobei die Mikroplatte 5-700 000 Vertiefungen pro cm² umfasst.

13. Verfahren nach einem der Ansprüche 2-4, wobei das Aussetzen unter Verwendung einer Durchflusszytometrievorrichtung erfolgt.

14. Verfahren nach einem vorhergehenden Anspruch, wobei die Zellen aus neoplastischen Zellen, menschlichen Leukämiezellen, menschlichen Myelomzellen und nichtmenschlichen Stammzellen ausgewählt sind.

15. Verfahren nach einem vorhergehenden Anspruch, wobei die Nukleinsäure aus DNA und RNA ausgewählt ist.

## Revendications

1. Méthode d'analyse génétique d'échantillons de cellule unique, comprenant les étapes
a) de fourniture d'au moins un échantillon de cellule unique contenu dans une microplaque comprenant au moins 5 puits/cm², où chaque échantillon de cellule unique est contenu dans un puits individuel de ladite microplaque, ledit échantillon de cellule unique étant choisi parmi une cellule unique et des cellules développées à partir d'une cellule unique ;
b) de lyse de chacun parmi ledit au moins un échantillon de cellule unique dans chacun parmi ledit ou lesdits puits individuels afin d'obtenir des acides nucléiques, tels que l'ADN et l'ARN, à partir dudit au moins un échantillon de cellule unique ;
c) d'amplification d'une portion génétiquement pertinente desdits acides nucléiques à l'aide d'une réaction en chaîne par polymérase (PCR) en utilisant au moins une amorce comprenant au moins un marqueur de capture afin d'obtenir un produit de PCR amplifié marqué dans chacun parmi ledit ou lesdits puits individuels ; et
d) d'analyse dudit produit de PCR amplifié marqué dans chacun parmi ledit ou lesdits puits individuels, où ladite analyse comprend la capture dudit produit de PCR dans chacun parmi lesdits puits individuels via ledit marqueur de capture vers un moyen de capture dans lesdits puits.

2. Méthode selon la revendication 1, dans laquelle ladite étape a) comprend
(i) la fourniture d'au moins un échantillon de cellule unique ; et
(ii) l'ensemencement d'au moins une cellule dudit échantillon de cellule unique dans au moins un puits de ladite microplaque ; et éventuellement
(iii) l'incubation de ladite microplaque dans des conditions de culture de cellules.

3. Méthode selon la revendication 2, dans laquelle ladite étape a) comprend
(iii) l'incubation de ladite microplaque dans des conditions de culture de cellules ; et
(iv) l'analyse d'au moins une propriété biologique pertinente dans au moins un puits de la microplaque.

4. Méthode selon la revendication 3, dans laquelle ladite analyse de l'étape a) (iv) comprend l'analyse d'une propriété choisie parmi la morphologie cellulaire, la prolifération cellulaire, les aptitudes de formation de clones, la différentiation cellulaire, les interactions cellule-cellule et le cycle de division cellulaire.

5. Méthode selon l'une quelconque des revendications précédentes, dans laquelle ladite PCR de l'étape c) est une PCR en phase solide.

6. Méthode selon l'une quelconque des revendications précédentes, dans laquelle lesdits moyens de capture sont constitués de billes magnétiques.

7. Méthode selon l'une quelconque des revendications précédentes, dans laquelle l'étape d) comprend en outre
(ii) la dénaturation et le lavage dudit produit de PCR capturé dans lesdits puits afin de fournir un ADN simple brin capturé sur ledit moyen de capture ;
(iii) l'analyse par voie génétique dudit ADN simple brin dans lesdits puits.

8. Méthode selon la revendication 7, dans laquelle ladite analyse génétique de l'étape (iii) comprend la détection de polymorphismes mononucléotidiques (SNP) par la réalisation d'une extension de base unique sur ledit ADN simple brin dans au moins l'un desdits puits.

9. Méthode selon l'une quelconque des revendications précédentes, dans laquelle ladite PCR de l'étape c) est réalisée en utilisant au moins une amorce comprenant au moins un marqueur de détection.

10. Méthode selon l'une quelconque des revendications 1-6, dans laquelle ladite analyse de l'étape d) comprend
(i) la détection de produits de PCR double brin dans au moins un parmi lesdits puits, éventuellement à l'aide d'un marqueur de détection.

11. Méthode selon la revendication 9 ou 10, dans laquelle ledit marqueur de détection est choisi dans le groupe constitué par les colorants fluorescents.

12. Méthode selon l'une quelconque des revendications précédentes, dans laquelle ladite microplaque comprend 5-700 000 puits/cm².

13. Méthode selon l'une quelconque des revendications 2-4, dans laquelle ledit ensemencement est réalisé à l'aide d'un appareil de cytométrie en flux.

14. Méthode selon l'une quelconque des revendications précédentes, dans laquelle les cellules sont choisies parmi les cellules néoplasiques, les cellules de leucémie humaine, les cellules de myélome humain, et les cellules souches non humaines.

15. Méthode selon l'une quelconque des revendications précédentes, dans laquelle ledit acide nucléique est choisi parmi l'ADN et l'ARN.
